(19) Europäisches Patentamt
European Patent Office
Office européen des brevets

(11) Veröffentlichungsnummer: **0 097 229**
**B1**

(12) **EUROPÄISCHE PATENTSCHRIFT**

(45) Veröffentlichungstag der Patentschrift: **10.06.87**

(51) Int. Cl.⁴: **C 08 B 37/08,** A 61 K 7/06

(21) Anmeldenummer: **83103606.6**

(22) Anmeldetag: **14.04.83**

(54) **Kosmetische Mittel auf der Basis von Chitosanderivaten, neue Chitosanderivate sowie Verfahren zur Herstellung dieser Derivate.**

(30) Priorität: **23.06.82 DE 3223423**

(43) Veröffentlichungstag der Anmeldung: **04.01.84 Patentblatt 84/1**

(45) Bekanntmachung des Hinweises auf die Patenterteilung: **10.06.87 Patentblatt 87/24**

(84) Benannte Vertragsstaaten: **DE GB IT SE**

(56) Entgegenhaltungen:
**EP-A-0 002 506**
**EP-A-0 020 183**
**EP-A-0 026 618**

(73) Patentinhaber: **Wella Aktiengesellschaft, Berliner Allee 65, D-6100 Darmstadt (DE)**

(72) Erfinder: **Lang, Günther, Dr., Frankensteiner Weg 1, D-6109 Mühltal 3 (DE)**
Erfinder: **Wendel, Harald, Grabengasse 3, D-6105 Ober- Ramstadt (DE)**
Erfinder: **Konrad, Eugen, Mecklenburger Strasse 101, D-6100 Darmstadt (DE)**

**EP 0 097 229 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1987

**Beschreibung**

Die Erfindung betrifft kosmetische Mittel zur Behandlung von Haaren oder der Haut mit einem Gehalt an neuen makromolekularen, vom Chitosan abgeleiteten Verbindungen, die in wäßrigen bzw. wäßrigalkoholischen Zubereitungen sowie gegebenenfalls mit weiteren Zusätzen zur Anwendung kommen.

Die Erfindung betrifft weiterhin neue Glyceryl-Chitosane, d. h. makromolekulare, vom Chitosan abgeleitete Verbindungen sowie Verfahren zu deren Herstellung.

Es ist bereits bekannt, in kosmetischen Mitteln, insbesondere für die Behandlung von Haaren, kationaktive Polymere, insbesondere Polymere, die quaternäre Ammoniumgruppen aufweisen, als Konditionierungsmittel einzusetzen. Auf Grund einer Wechselwirkung zwischen ihren Ammoniumgruppen und den anionischen Gruppen des Haares besitzen die kationaktiven Polymere eine große Affinität zur Keratinfaser.

Es wurde festgestellt, daß der Einsatz derartiger kationaktiver Polymere in solchen kosmetischen Mitteln zahlreiche Vorteile ergibt; die Entwirrung der Haare sowie deren Behandlung wird erleichtert, und weiterhin wird dem Haar Sprungkraft und Glanzwirkung verliehen. Durch die Affinität zum Keratin neigen diese Polymere jedoch bei wiederholter Anwendung zur Ansammlung auf den Haaren, so daß diese schwerer werden, was im Endeffekt unerwünscht ist.

Weiterhin ergeben sich bei synthetischen Polymeren Probleme wegen der physiologischen Wirkung von eventuell vorhandenen Monomerspuren, die nur schwer aus dem Polymer entfernbar sind.

Man hat bereits versucht, diese vorerwähnten Nachteile dadurch zu beheben, daß wasserlösliche Salze des Chitosans, eines durch Entacetylierung von Chitin herstellbaren Polyglucosamins, in derartigen kosmetischen Mitteln Anwendung finden. In diesem Zusammenhang wird Bezug genommen auf die eigene europäische Patentschrift 0 002 506 sowie die eigene deutsche Patentschrift 26 27 419.

In gleicher Weise wie bei der Mehrzahl der kationaktiven Polymere mit quaternären Gruppierungen ergibt Chitosan ebenfalls häufig den Nachteil, daß es mit den anionaktiven oberflächenaktiven Agentien, die üblicherweise in kosmetischen Mitteln zur Behandlung von Haaren, insbesondere in Shampoos, Anwendung finden, wenig verträglich ist. Es ist daher notwendig, das Chitosan in separaten Behandlungen, nämlich vor und/oder nach der Shampoonierung zur Einwirkung zu bringen.

Das Chitosan erweist sich weiterhin in neutralem und alkalischem Medium als praktisch unlöslich, so daß seine Anwendung beispielsweise in alkalischen Dauerwellmitteln oder Haarfärbemitteln nicht möglich ist.

Aufgabe der Erfindung ist es, diese Nachteile zu beseitigen.

Bei Fortführung der Untersuchungen mit Chitosan und den davon abgeleiteten Verbindungen wurde nunmehr gefunden, daß gewisse Chitosan-Derivate, und zwar speziell Glyceryl-Chitosane, die vorstehend aufgeführten Nachteile nicht aufweisen.

Mit Glyceryl-Chitosan bzw. seinen Salzen mit organischen oder anorganischen Säuren lassen sich somit kosmetische Mittel zur Behandlung von Haaren oder Haut herstellen, die sich durch überraschend vorteilhafte Eigenschaften auszeichnen und die gekennzeichnet sind durch einen Gehalt an Glyceryl-Chitosan, und zwar makromolekularen, vom Chitosan abgeleiteten Verbindungen, bestehend aus:

a) 4-40 Mol% Einheiten der Formel (I)

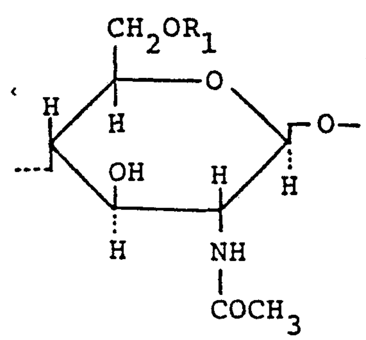

und

b) 60-96 Mol% Einheiten der Formel (II)

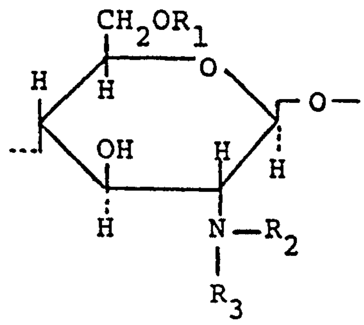

- worin $R_1$, $R_2$ und $R_3$ gleich oder verschieden sind und

—H ,

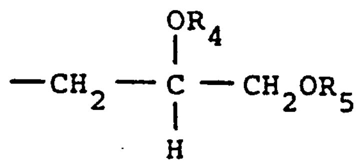

oder

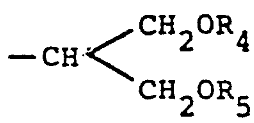

darstellen, wobei $R_4$ und $R_5$ gleich oder verschieden sind und die Substituenten

—H ,

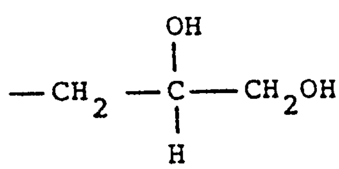

,

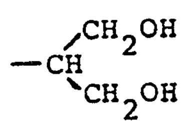

oder

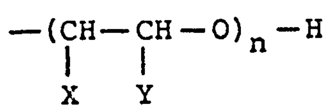

darstellen, worin entweder X=H und Y=$CH_2OH$ bedeuten oder Y=H und X=$CH_2OH$ bedeuten und n = 2 bis 5 ist, mit der Maßgabe, daß bei mindestens der Hälfte der Einheiten der Formel II $R_1$, $R_2$ und $R_3$ nicht gleichzeitig H sind, bzw. dessen Salze mit organischen oder anorganischen Säuren.

Die Salze der erfindungsgemäßen Glyceryl-Chitosane können beispielsweise durch Neutralisierung der Aminogruppen der Glyceryl-Chitosane mit Säuren erhalten werden. Gemäß der vorliegenden Erfindung sind

jedoch nur solche Salze verwendbar, die in Wasser löslich sind. Geeignete Salze sind z. B. solche, die mit den Säuren Milchsäure, Ameisensäure und Essigsäure gebildet werden.

Die erfindungsgemäßen Glyceryl-Chitosan enthaltenden Mittel eignen sich ganz allgemein zur Behandlung der Haut und/oder der Haare. Sie können beispielsweise vorliegen als Haar- und/oder Körperwaschmittel, Tönungsshampoos, Frisiercremes, Frisierlotionen, Fönlotionen, Mittel zur Festigung der Frisur, Waschlotionen, Haarkuren, Mittel gegen Kopfschuppen, Mittel zur permanenten Haarverformung, Mittel zur Färbung oder Entfärbung von Haaren, Mittel zum Auftragen vor oder nach der Haarfärbung und als kosmetische Mittel zur Pflege, zum Schutz oder zur Reinigung der Haut wie Gesichtswässer, Rasierwässer, Feuchthaltecremes, Coldcremes, Körperlotionen, Sonnenschutzmittel oder auch Make-up-Präparate wie Schminkcremes und Rouges.

Die kosmetischen Mittel gemäß der vorliegenden Erfindung können zusätzlich zu dem neuen Wirkstoff Glyceryl-Chitosan alle diejenigen Bestandteile enthalten, die in Haar- und Hautbehandlungsmitteln üblicherweise eingesetzt werden, insbesondere anionische, kationische, amphotere, zwitterionische oder nichtionische oberflächenaktive Tenside, Schaumsynergisten, Stabilisatoren, Sequestrierungsmittel, Pigmente, Verdicker, Emulgatoren, Pufferstoffe, Konservierungsmittel, Farbstoffe, Parfümöle, bekannte kosmetische Polymere wie anionische, nichtionische, kationische oder amphotere Polymere, Naturstoffe, kosmetische Öle, Fettalkohole, Wachse, Schaumstabilisatoren, Wirkstoffe gegen Kopfschuppen, Reduktionsmittel und Treibgase.

Die erfindungsgemäßen kosmetischen Mittel weisen in bevorzugter Weise einen pH-Wert von 2 bis 11 auf und können in Form wäßriger Zubereitungen, wäßrig-alkoholischer Zubereitungen, z. B. mit einem Alkohol mit 1 bis 4 Kohlenstoffatomen, als Lösungen, als Cremes, als Gele, als Dispersionen oder als Emulsionen vorliegen. Ebenfalls ist es möglich, diese Mittel mit Hilfe eines Zerstäubers bzw. anderer geeigneter Sprühvorrichtungen oder im Gemisch mit üblichen Treibgasen aus einem Druckbehälter zu versprühen.

Wenn es sich bei den erfindungsgemäßen kosmetischen Mitteln um Mittel zur Festigung der Frisur, wie flüssige Haarfestiger oder Haarsprays, handelt, dann liegen sie üblicherweise als wäßrige oder wäßrigalkoholische Lösungen vor, die durch einen Gehalt an Glyceryl-Chitosan aus Einheiten der vorstehend genannten Formel I und II bzw. dessen löslichen Salzen mit organischen oder anorganischen Säuren gekennzeichnet sind. Hierbei kann das Glyceryl-Chitosan selbst als filmbildendes bzw. festigendes Harz eingesetzt werden. Es können jedoch auch zusätzlich andere filmbildende natürliche oder synthetische Polymere in dem erfindungsgemäßen Haarfestigungsmittel enthalten sein. Als natürliche Polymere kommen beispielsweise Schellack, Alginate, Gelatine, Pektine und Cellulosederivate in Betracht. Von den synthetischen Polymeren finden z. B. Polyvinylpyrrolidon, Polyvinylacetat, Polyacrylverbindungen, wie Acrylsäure- oder Methacrylsäurepolymerisate, basische Polymerisate von Estern aus Acrylsäure oder Methacrylsäure mit Aminoalkoholen bzw. die Salze oder Quaternisierungsprodukte dieser basischen Polymerisate, Polyacrylnitril sowie Co- oder Terpolymerisate aus derartigen Verbindungen, beispielsweise Polyvinylpyrrolidon-Vinylacetat, Verwendung.

Als Alkohole kommen insbesondere die für kosmetische Zwecke üblicherweise verwendeten niederen Alkohole wie Äthylalkohol und Isopropylalkohol in Betracht.

Die erfindungsgemäßen Mittel zur Festigung der Frisur können weiterhin die üblichen Zusätze wie beispielsweise Parfümöle, Bakterizide oder Fungizide, kämmbarkeitsverbessernde Substanzen usw. enthalten.

Die erfindungsgemäßen Mittel zur Festigung der Frisur können gegebenenfalls durch einen Gehalt an kosmetischen Farbstoffen das Haar gleichzeitig färben oder tönen. Derartige Präparate sind u. a. als Farbfestiger oder Tönungsfestiger im Handel bekannt. Sie enthalten zusätzlich übliche für Haarfestiger bekannte Farbstoffe wie beispielsweise aromatische Nitrofarbstoffe (z. B. 1,4-Diamino-2-nitrobenzol), Azofarbstoffe (z. B. C.I. Acid Brown 4), Anthrachinonfarbstoffe (z. B. C.I. Disperse Violett 4) und Triphenylmethanfarbstoffe (z. B. C.I. Basic Violett 1), wobei die Farbstoffe dieser Klassen je nach der Art ihrer Substituenten sauren, nichtionogenen oder basischen Charakter haben können. Ihre Gesamtkonzentration in diesen Präparaten beträgt üblicherweise etwa 0,01 bis 2,0 Gew. %.

Die erfindungsgemäßen Mittel zur Festigung der Frisur weisen bei gleich guter Festigung das Haares gegenüber üblichen Mitteln eine besonders gute Kämmbarkeit und einen guten Griff des Haares im nassen Zustand sowie einen besonders angenehmen Griff des Haares im getrockneten Zustand auf.

Wenn die erfindungsgemäßen Mittel Haarwaschmittel darstellen, liegen sie in Form wäßriger Lösungen oder Emulsionen vor und enthalten neben dem Glyceryl-Chitosan zumindest ein anionisches, kationisches, nichtionisches oder amphoteres Tensid.

In diesem Haarwaschmittel liegt die Konzentration des Tensides im allgemeinen zwischen 3 und 50 Gew. % und vorzugsweise zwischen 3 und 20 Gew. %, bezogen auf das Gesamtgewicht des Mittels, wobei der pH-Wert im allgemeinen zwischen 3 und 9 und vorzugsweise zwischen 4 und 7 liegt.

Die erfindungsgemäßen Mittel, die in Form von Haarwaschmitteln vorliegen, enthalten im allgemeinen verschiedene Zusatzstoffe, insbesondere Parfüms, Konservierungsstoffe, Verdicker, Schaumstabilisatoren, Puffersubstanzen, kosmetische Harze, Pigmente und Farbstoffe.

Unter den Schaumstabilisatoren können die Fettamide und insbesondere die Mono- oder Diäthanolamide von Copra-Fettsäuren, Lauryl- oder Ölsäuremono- oder -diäthanolamid, die zweckmäßigerweise in Mengen von 1 bis 10 und vorzugsweise 1 bis 3 Gew. %, bezogen auf das Gesamtgewicht des Mittels Verwendung finden, angeführt werden.

Unter den Verdickern können insbesondere die Acrylpolymere und die Cellulosederivate wie

Carboxymethylcellulose, Hydroxypropylmethylcellulose, Hydroxyäthylcellulose angeführt werden. Die Verdicker liegen im allgemeinen in einem Anteil von 0,1 bis 5 Gew. % vor.

Unter den Tensiden oder oberflächenaktiven Agentien, die in Kombination mit den neuen Glyceryl-Chitosanen verwendet werden, können beispielsweise die folgenden genannt werden:

a) die anionischen oberflächenaktiven Agentien, wie beispielsweise die Alkali- oder Erdalkalisalze oder Alkanolamine von Alkansulfonaten, Alkylsulfaten und Alkyläthersulfaten, die $C_{12}$ - $C_{18}$-Alkyl- und insbesondere $C_{12}$ - $C_{14}$-Alkyl-Sulfatnatriumsalze oder Triäthanolaminsalze, die Natrium- oder Triäthanolaminsalze von Lauryl- oder Tetradecyläthersulfaten, das Dinatriumsalz des Sulfosuccinhalbesters von Alkanolamiden, die Seifen und die Polyäthercarbonsäuren;

b) die nichtionischen oberflächenaktiven Agentien, wie beispielsweise oxäthylierte Fettalkohole mit 12 bis 18 Kohlenstoffatomen, z. B. mit bis zu 40 Mol Äthylenoxid Pro Mol Fettalkohol, oxäthylierter Laurin-, Tetradecyl-, Cetyl-, Olein-, Palmitin- und Stearinalkohol, allein oder im Gemisch, die Fettalkohole von oxäthyliertem Lanolin oder oxäthyliertes Lanolin; Polyglyceryläther von gesättigten oder ungesättigten Fettalkoholen und Alkylphenolen mit 8 bis 30 Kohlenstoffatomen im Alkylrest und 1 bis 10 Glyceryleinheiten im Molekül sowie Fettsäurealkanolamide;

c) die kationischen oberflächenaktiven Agentien, wie beispielsweise das Dilauryldimethylammoniumchlorid, die Chloride oder Bromide von Alkyldimethylbenzylammonium, die Chloride oder Bromide von Alkyltrimethylammonium, beispielsweise Cetyltrimethylammoniumchlorid oder -bromid, Tetradecyltrimethylammoniumchlorid oder -bromid, die Alkyldimethylhydroxyäthylammoniumchloride oder -bromide, die Dialkyldimethylammoniumchloride oder -bromide, Alkylpyridiniumsalze, beispielsweise Cetylpyridiniumchlorid, die Alkylamidäthyltrimethylammoniumäthersulfate, Imidazolinderivate, Verbindungen mit kationischem Charakter wie Aminoxide, beispielsweise Alkyldimethylaminoxide oder Alkylaminoäthyldimethylaminoxide;

d) die amphoteren oder zwitterionischen oberflächenaktiven Agentien wie beispielsweise die Carboxylderivate von Imidazol, die N-Alkylbetaine, die N-Alkylsulfobetaine, die N-Alkylaminobetaine, die N-Alkylaminopropionate, die Alkyldimethylammoniumacetate bzw. die $C_{12}$ - $C_{18}$ - Alkyldimethylcarboxymethylammoniumsalze.

Die erfindungsgemäßen kosmetischen Mittel können auch Cremes oder Lotionen zur Verwendung als Haarkur oder Hautpflegemittel darstellen. Sie liegen dann meist in Form von Öl-in-Wasser- oder Wasser-in-Öl-Emulsionen oder Suspensionen vor und enthalten zusätzlich zu den neuen Glyceryl-Chitosanen kationische, nichtionogene, amphotere oder anionische Emulgatoren sowie als Bestandteil der Ölphase z. B. Fettalkohole, Fettsäureester- oder -amide, weiterhin Parfümöle, Vaseline, Wollfettalkohol oder feste oder flüssige Paraffine.

Wenn die erfindungsgemäßen Mittel Haartönungs- oder Haarfärbemittel darstellen, so liegen sie ebenfalls bevorzugt in Form von Cremes oder Lotionen vor und enthalten zusätzlich übliche Haarfarbstoffe aus der Gruppe der aromatischen Nitrofarbstoffe, Azofarbstoffe, Anthrachinonfarbstoffe, Triphenylmethanfarbstoffe oder auch Oxidationsfarbstoffe, beispielsweise aus der Gruppe der aromatischen Diamide bzw. Aminophenole. Weiterhin können diese Mittel gegebenenfalls Alkalisierungsmittel, Antioxidantien sowie weitere für solche Mittel übliche kosmetische Zusätze und Hilfsstoffe enthalten.

Die erfindungsgemäßen Mittel können auch Dauerverformungsmittel oder Fixiermittel für Haare darstellen. Sie enthalten dann zusätzlich zu den genannten Glyceryl-Chitosanen Reduktionsmittel, wie Thioglykolsäure, Thiomilchsäure, Ammoniumsulfit bzw. Oxidationsmittel, wie Wasserstoffperoxid oder Natriumbromat sowie gegebenfalls Alkalisierungsagentien bzw. Peroxidstabilisatoren, z. B. Phosphorsäure, und andere kosmetische Hilfsstoffe und Zusatzstoffe wie beispielsweise Parfümöle, Riechstoffe, Pflegestoffe und Farbstoffe.

Der Gehalt der erfindungsgemäßen kosmetischen Mittel an Glyceryl-Chitosan liegt zweckmäßig bei 0,05 bis 10 Gew. %, vorzugsweise bei 0,05 bis 3,0 Gew. %.

Die in den erfindungsgemäßen kosmetischen Mitteln enthaltenen neuen Chitosan-Derivate leiten sich von Chitosan ab, einem Material, das durch Deacetylierung von Chitin, einem natürlich vorkommenden Acetylglucosamin, erhalten wird.

Das Chitosan ist im neutralen und alkalischen Medium unlöslich, es bildet jedoch auf Grund seiner chemischen Natur im sauren Medium mit organischen und anorganischen Säuren Salze, die beispielsweise in der Papier- und Textilindustrie als Additive Verwendung finden sowie weiterhin als Koagulanzien für Suspensionen, als Geliermittel für Schwermetalle sowie in der Medizin und in der Kosmetik benutzt werden. (Siehe in diesem Zusammenhang die Veröffentlichung von Muzarelli: "Chitin", Pergamon Press, 1977).

Es sind auch bereits einige wasserlösliche Chitosanderivate bekannt, beispielsweise Carboxymethylchitosan, Sulfoäthylchitosan (siehe Nud'ga, Plisko und Darnilov, Zhur. Prikl. Khim. 47, 872 - 875). Diese wasserlöslichen Chitosanderivate sind jedoch in ihrem ionischen Charakter verändert oder aber physiologisch bedenklich (Epichlorhydrinchitosan, Veröffentlichung von Noguchi, Arato und Komai; Kogyo Kagaku Zasshi 72, 769 - 799 und japanische Patentanmeldung Nr. 43-39 322, H. Haga).

Diese vorerwähnten Stoffe fordern weiterhin für ihre technische Herstellung recht aufwendige Verfahren.

Aus der Veröffentlichung von A. Kleemann, R. Wagner "Glycidol,: Properties, Reactions, Applications", Hüthig Verlag 1981, S. 84ff., ist weiterhin die Umsetzung von Glycidol mit primären Aminen im Prinzip bekannt. Reaktionen des Glycidols mit aminogruppenhaltigen Polymeren wurden jedoch bisher nicht beschrieben.

Es wurde nur mehr gefunden, daß sich durch Umsetzung von Chitosan mit Glycidol auf einfache Weise neue Chitosanderivate herstellen lassen, die bei geeignetem Substitutionsgrad gut wasserlöslich sind und hervorragende Filmeigenschaften besitzen.

Bei dieser Umsetzung reagiert in erster Linie die Aminogruppe des Glucosaminbausteins mit dem Glycidol (1,2-Epoxypropanol-3 oder Glycerinanhydrid); darüber hinaus kann jedoch auch eine Substitution an der primären Alkoholgruppe in 2-Stellung erfolgen.

Die neuen makromolekularen, vom Chitosan abgeleiteten Verbindungen sind dadurch gekennzeichnet, daß sie bestehen aus

a) 4-40 Mol% Einheiten der Formel (I)

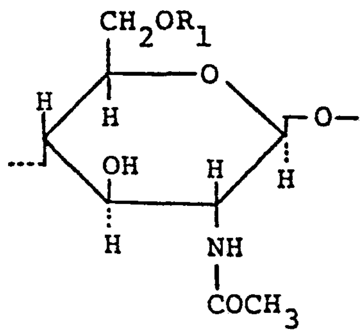

b) 60-96 Mol% Einheiten der Formel (II)

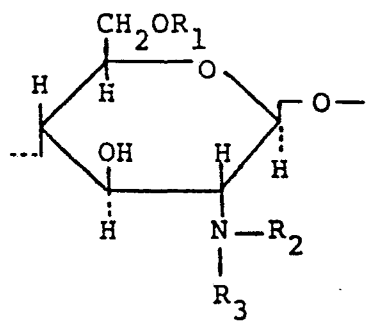

- worin $R_1$, $R_2$ und $R_3$ gleich oder verschieden sind und

$-H$ ,

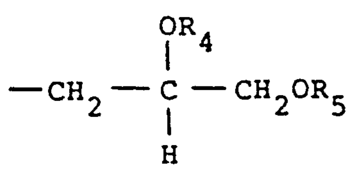

oder

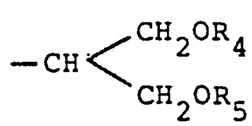

darstellen, wobei $R_4$ und $R_5$ gleich oder verschieden sind und die Substituenten

$-H$ ,

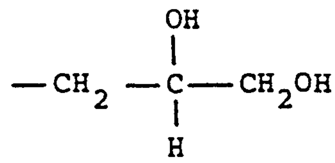

,

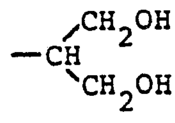

oder

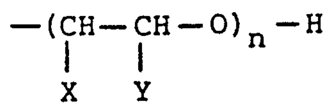

darstellen,
wobei entweder X = H und Y = $CH_2OH$ bedeuten oder Y = H und X = $CH_2OH$ bedeuten und n = 2 bis 5 ist, mit der Maßgabe, daß bei mindestens der Hälfte der Einheiten der Formel II $R_2$ und $R_3$ nicht gleichzeitig H sind, sowie deren wasserlöslichen Salze mit organischen oder anorganischen Säuren.

Die neuen Glyceryl-Chitosane werden erfindungsgemäß hergestellt, indem ein Chitosan, bestehend aus zu 60 - 96 % eentacetyliertem Chitin, mit Glycidol (1,2-Epoxypropanol-3) in geeignetem Verhältnis zur Umsetzung bringt.

Hierzu wird zweckmäßig das Chitosan in feingepulverter Form eingesetzt. Die Umsetzung selbst kann bei Temperaturen zwischen 10 und 100° C, vorzugsweise zwischen 50 und 80° C erfolgen und diese Temperatur wird zweckmäßig unter Rühren 2 bis 100 Stunden beibehalten. Die Reaktion kann mit oder ohne saure oder basische Katalysatoren in Gegenwart von Lösungsmitteln oder ohne Lösungsmittel erfolgen.

Vorzugsweise wird die Reaktion in Gegenwart von Wasser durchgeführt. Es hat sich dabei als vorteilhaft erwiesen, ohne zusätzliche Katalysatoren zu arbeiten, wobei das Verhältnis von Chitosan zu Wasser zwischen 1: 0,05 und 1 : 100 und das Verhältnis von Chitosan zu Glycidol (bezogen auf die Mole von substituierbaren Aminogruppen am Chitosan) zwischen 1 : 0,5 und 1 : 30, vorzugsweise zwischen 1 : 1 und 1 : 10, liegt.

Obwohl die Durchführung der Reaktion in Gegenwart von Wasser bevorzugt ist, kann sie auch unter Verwendung anderer Lösungsmittel, in denen mindestens eines der Reaktionsprodukte löslich ist, durchgeführt werden. Beispiele für solche Lösungsmittel sind Alkohole wie Äthanol, Methanol, Glykol und Glycerin sowie Ketone, beispielsweise Methyläthylketon und Aceton.

Gemäß einer anderen Ausführungsform des erfindungsgemäßen Verfahrens zur Herstellung der Glyceryl-Chitosane werden bei der Umsetzung zusätzlich organische oder anorganische Säuren oder Basen als Katalysatoren zugesetzt.

Für einen Einsatz als Katalysatoren geeignete Säuren sind zum Beispiel Salzsäure, Milchsäure und Ameisensäure. Beispiele für geeignete Basen sind Trialkylamine wie beispielsweise Trimethylamin, Triäthylamin oder Trialkylolamine sowie Alkalihydroxide und Erdalkalihydroxide. Es ist grundsätzlich auch möglich, von wasserlöslichen Salzen des Chitosans, beispielsweise Chitosanlaktat, Chitosanacetat, Chitosanhydrochlorid usw. auszugehen. Hierbei kann jedoch als Nebenprodukt eine größere Menge der Glycerinester der verwendeten Säure entstehen, so daß die Reinigung der entstandenen Reaktionsprodukte erschwert wird.

Die Aufarbeitung der Reaktionsgemische kann beispielsweise in der Weise erfolgen, daß man das Lösungsmittel und gegebenenfalls überschüssiges Glycidol im Vakuum aus der Reaktionsmischung abdestilliert.

Bei der Herstellung von wasserlöslichen Glyceryl-Chitosanen kann das Reaktionsprodukt in bevorzugter Weise in einem Überschuß an Wasser gelöst und durch Filtration oder Zentrifugieren vom nicht-löslichen Reaktionsrückstand abgetrennt werden. Als weitere Schritte zur Reinigung der Reaktionsprodukte können die wäßrigen Lösungen dialysiert werden und/oder gegebenenfalls nach Einengen der wäßrigen Lösungen durch Ausfällen in Aceton, Alkoholen oder anderen organischen Lösungsmitteln isoliert werden.

Gemäß einer besonders vorteilhaften Ausführungsform des erfindungsgemäßen Herstellungsverfahrens wird als Ausgangsmaterial durch Umfällen und Tiefgefrieren strukturell modifiziertes Chitosan eingesetzt. Mit einem solchen Ausgangsmaterial verläuft die Reaktion in besonders vorteilhafter Weise und in besonders guter

Ausbeute.

Anhand der nachfolgenden Beispiele wird das erfindungsgemäße Verfahren zur Herstellung der neuen Glyceryl-Chitosane näher erläutert.

**Beispiel 1**

12 g Chitosan mit einer Grenzviskositätszahl η = 140 ml/g (ermittelt im DIN-Ubbelohde Viskosimeter mit 0,2 m Essigsäure und 0,1 m Natriumacetat als Lösungsmittel) und einem freien Amin-Gehalt von 86 % wurden mit 24 ml Glycidol (Molverhältnis 1 : 4,5) umgesetzt.

Der Gehalt an freiem Amin wurde durch potentiometrische Titration in wasserfreier Essigsäure als Lösungsmittel bestimmt. Titriert wurde mit 0,1 m Perchlorsäure in wasserfreier Essigsäure.

Die Reaktion wurde in einem Zweihalsrundkolben bei einer Temperatur im Bereich von 20 - 60°C durchgeführt. Reaktionsdauer: 30 Stunden.

Danach hatte sich der Ansatz so verdickt, daß er nicht mehr rührbar war.

Das Reaktionsgemisch wurde zur Lösung des wasserlöslichen Anteils in 1 l Wasser gegeben. Der unlösliche Rückstand wurde durch Filtrieren bzw. Zentrifugieren abgetrennt. Die wäßrige Lösung wurde in Vakuum auf etwa 200 ml eingeengt und danach das Reaktionsprodukt in 2 l Aceton ausgefällt. Nach Trocknung im Vakuum bei 50°C fielen 11,5 g des klar wasserlöslichen Chitosanderivates an.

**Kenndaten des Reaktionsgroduktes:**

| | |
|---|---|
| Grenzviskositätszahl: | η = 67 ml/g |
| Titrierbarer Stickstoff: | 3,3 mmol/g |
| Substitutionsgrad daraus ermittelt: | 1,44 |

Die wäßrige Lösung bildet bei dem Eintrocknen einen klaren elastischen Film.

**Beispiel 2**

100 g Chitosan (η = 140 ml/g; freies Amin 86 %) wurden in einem Doppelmantelrührgefäß mit 450 ml Glycidol (Molverhältnis 1 : 9) versetzt und 96 Stunden bei Temperaturen im Bereich von 20 bis 60°C gerührt. Das Reaktionsprodukt wurde in ca. 5 l Wasser gegeben, und der unlösliche Rückstand wurde abzentrifugiert.

Die wäßrige Lösung wurde eingeengt und das Reaktionsprodukt durch Ausfällen in Aceton isoliert.

Nach dem Trocknen im Vakuum bei 50°C wurde eine Ausbeute von 60 g erhalten. Ein Teil der Substanz wurde wieder in Wasser gelöst und 1 Woche dialysiert. (Dialyseschlauch Trenngrenze 1000).

**Kenndaten des Reaktionsproduktes:**

| | a) nicht dialysiert, | b) dialysiert |
|---|---|---|
| Grenzviskosität (ml/g) : | 44 | 33 |
| Titrierbarer Stickstoff: | 2,7 mmol/g | 3,1 mmol/g |
| Substitutionsgrad daraus: | 2,4 | 1,7 |

Die wäßrigen Lösungen ergaben beim Eintrocknen elastische Filme. Die Wasserdampfaufnahme beträgt bei 80 % Luftfeuchte gegenüber 30 % Luftfeuchte 10 Gew. %.

Die Pendelhärte nach Koenig lag bei 185 s.

**Beispiel 3**

Die nachstehenden vier Versuche veranschaulichen den Einfluß von Lösungsmittel und Katalysator.

Hierzu wurde ein Chitosan mit den Kenndaten η = 140 ml/g: freies Amin 86 % eingesetzt.

a) 10 g Chitosan wurden über Nacht in 50 ml Wasser vorgequollen und danach abgesaugt. Das feuchte Chitosan wurde zur Reaktion eingesetzt.

b) 10 g Chitosan wurden über Nacht in 50 ml Wasser vorgequollen und mit dem Wasser als Lösungsmittel zur

Reaktion eingesetzt.

c) 10 g Chitosan wurden mit 10 g 10 %iger Milchsäure und 40 ml Wasser über Nacht angequollen und zur Reaktion eingesetzt.

d) 10 g Chitosan wurden in einer Lösung bestehend aus 100 ml Äthylenglykol und 1 ml Tripropylamin zur Reaktion eingesetzt.

Die Reaktion wurde mit je 40 ml Glycidol bei Raumtemperatur durchgeführt. Die Hälfte der Proben wurden nach 1 Tag aufgearbeitet, der Rest nach 5 Tagen.

| Ausbeute nach: | a | b | c | d |
|---|---|---|---|---|
| 1 Tag | 1 g | 1,5 g | 2 g | - |
| 5 Tagen | 6 g | 4 g | 3,5 g | 6,5 g |

**Kenndaten der Reaktionsprodukte:**

| | | a | b | c | d |
|---|---|---|---|---|---|
| Substitutionsgrad aus titrierbarem N ermittelt: | 1 Tag | 1,8 | 2,2 | 2,5 | - |
| | 5 Tage | 1,7 | 2,2 | 2,5 | 1,6 |
| Grenzviskositätszahl η in ml/g: | 5 Tage | 36 | 30 | 52 | 51 |

**Beispiel 4**

In diesem Beispiel wurden Chitosanproben mit unterschiedlichem Entacetylierungsgrad eingesetzt:

Chitosan a) η = 140 ml/g
freie $NH_2$ = 86 %

b) η = 1100 ml/g
freie $NH_2$ = 62 %

Je 10 g Chitosan wurden mit 13,3 ml Glycidol umgesetzt. Reaktionsdauer: 4 Tage bei Raumtemperatur.

Ausbeuten: a) 6,5 g b) 3,5 g

**Kenndaten der erhaltenen Reaktionsprodukte:**

| | a) | b) |
|---|---|---|
| Grenzviskositätszahl η: | 69 ml/g | 201 ml/g |
| Substitutionsgrad aus titrierbarem N ermittelt: | 1,8 | 1,9 |

Die wäßrigen Lösungen ergaben klare elastische Filme. Die Wasserdampfaufnahme beträgt bei 80 % Luftfeuchte gegenüber 30 % Luftfeuchte 10 Gew. %.

Pendelhärte nach Koenig: a) 170 s b) 160 s.

**Beispiel 5**

40 g Chitosan (η = 650 ml/g; freies Amin 75 %) wurden bei diesem Versuch mit 67 g Glycidol (Molverhältnis 1 : 3) umgesetzt. Es wurden zuerst 44 g Glycidol zugesetzt und die Mischung 24 Stunden bei Raumtemperatur gerührt. Danach wurden die restlichen 23 g Glycidol zugegeben und noch 72 Stunden bei Raumtemperatur gerührt. Anschließend wurde das Reaktionsprodukt in Wasser gelöst und die Lösung mit Salzsäure auf pH 4,2 eingestellt.

Diese Lösung wurde nun geteilt. Ein Teil wurde 1 Woche dialysiert, der Rest wurde wie in Beispiel 1 beschrieben aufgearbeitet.

**Kenndaten der Reaktionsprodukte:**

| | dialysiert | nicht dialysiert |
|---|---|---|
| Ausbeuten: | 22,7 g | 32 g |
| Grenzviskositätszahl η: | 203 ml/g | 231 ml/g |
| Substitutionsgrad aus titrierbarem N ermittelt: | 3,1 | 7,1 |

Die wäßrigen Lösun9en ergaben beim Eintrocknen klare elastische Filme. Die Wasserdampfaufnahme lag bei 9 %; die Pendelhärte nach Koenig bei 150 s.

**Beispiel 6**

10 g Chitosan (η = 140 ml/g; freies Amin 86 %) wurden in Salzsäure gelöst und danach filtriert. Das Chitosan wurde dann mit Natronlauge aus dem Filtrat wieder ausgefällt. Der fein disperse Niederschlag wurde abgesaugt und über Nacht tiefgefroren. Die Probe wurde aufgetaut, nochmals abgesaugt und mit 17 g Glycidol bei 80° C zur Reaktion gebracht. Nach 3 Stunden war der Ansatz klar gelöst und hoch viskos.

Die Substanz wurde dann in Aceton ausgefällt und bei 50° C im Vakuum getrocknet. Die Ausbeute betrug 11 g.

**Kenndaten des Reaktionsproduktes:**

| | |
|---|---|
| Grenzviskositätszahl η: | 100 ml/g |
| Substitutionsgrad aus titrierbarem Stickstoff ermittelt: | 2,2 |

Nachfolgend werden Beispiele für kosmetische Mittel auf der Basis der neuen erfindungsgemäßen Glyceryl-Chitosan-Verbindungen gegeben:

**Beispiel 7**

| Haarfestiger | |
|---|---|
| 0,6 g | Glyceryl-Chitosan (η = 33 ml/g, Substitutionsgrad = 1,7) |
| 73,8 g | Wasser |
| 25,0 g | Isopropanol |
| 0,4 g | 10 %ige Ameisensäure |
| 0,2 g | Parfümöl |
| 100,0 g | |

20 ml dieser Lösung wurden auf gewaschenes, handtuchtrockenes Haar verteilt, das Haar in üblicher Weise zur Frisur eingelegt und getrocknet. Bei guter Festigungswirkung zeigte das Haar, im Vergleich zu einem Haarfestiger auf der Basis von Chitosan/Ameisensäure, einen angenehmeren und weicheren Griff.

**Beispiel 8**

| Tönungsfestiger | |
|---|---|
| 1,00 g | Glyceryl-Chitosan (η= 201 ml/g, Substitutionsgrad = 1,9) |
| 1,00 g | Milchsäure |
| 0,10 g | Cetyltrimethylammoniumchlorid |
| 0,05 g | Acid Brown 4 (C.I. 41 805) |
| 97,85 g | Wasser |
| 100,00 g | |

20 ml dieser Lösung wurden auf dem gewaschenen, handtuchtrockenen Haar verteilt und das Haar in

üblicher Weise eingelegt und getrocknet. Das Haar zeigte anschließend eine leichte Rot-Braunfärbung.

**Beispiel 9**

Tönungsfestiger

| | |
|---|---|
| 0,60 g | Glyceryl-Chitosan (η = 203 ml/g, Substitutionsgrad = 3,1) |
| 0,15 g | 1,4-Di(β-hydroxyäthylamino)-2-nitro-5-chlorbenzol |
| 25,00 g | Äthanol |
| 74,25 g | Wasser |
| 100,00 g | |

20 ml dieser Lösung wurden auf die gewaschenen, handtuchtrockenen Haare gegeben, sodann das Haar eingelegt und getrocknet. Die Haare waren rot-violett gefärbt und getestigt.

**Beispiel 10**

Anionisches Haarwaschmittel

| | |
|---|---|
| 1,00 g | Glyceryl-Chitosan (η = 52 ml/g, Substitutionsgrad = 2,5) |
| 40,00 g | Laurylalkoholdiglykoläthersulfat-Natriumsalz, 28 %ige wäßrige Lösung |
| 4,00 g | Natriumchlorid |
| 0,05 g | Farbstoff |
| 54,85 g | Wasser |
| 0,10 g | Formaldehyd, 25 %ige wäßrige Lösung |
| 100,00 g | |

Es wurde ein klares Shampoo erhalten. Das damit gewaschene Haar war hinsichtlich Griff, Glanz und Kämmbarkeit ausgezeichnet konditioniert.

**Beispiel 11**

Amphoteres, tönendes Haarwaschmittel

| | |
|---|---|
| 2,00 g | Glyceryl-Chitosan (η = 67 ml/g, Substitutionsgrad = 1,44) |
| 40,00 g | Dimethyl-carboxymethylen-propylenamido-stearatbetain, 35 %ige wäßrige Lösung |
| 5,06 g | Ameisensäure, 10 %ig |
| 3,50 g | Kokosfettsäurediäthanolamid |
| 1,00 g | Pikraminsäure (C.I. 76 540), 1 %ige wäßrige Lösung |
| 48,44 g | Wasser, vollentsalzt |
| 100,00 g | |

Mit etwa 15 bis 20 g wurde das Haar einshampooniert. Nach einer Einwirkungszeit von 5 bis 10 Minuten spülte man mit Wasser aus. Das Haar war gelb-orange getönt und ausgezeichnet konditioniert.

**Beispiel 12**

Haarkurmittel, kationisch

| | |
|---|---|
| 0,30 g | Glyceryl-Chitosan ($\eta$ = 44 ml/g, Substitutionsgrad = 2,4) |
| 4,00 g | Cetylstearylalkohol |
| 1,48 g | Milchsäure, 10 %ig |
| 2,50 g | Kokos(pentaäthoxy)methylammoniumchlorid |
| 1,00 g | Sorbitanmonopalmitat mit 20 Mol Äthylenoxyd |
| 90,72 g | Wasser, vollentsalzt |
| 100,00 g | |

**Beispiel 13**

Haarkurmittel, gelförmig

| | |
|---|---|
| 2,1 g | Glyceryl-Chitosan ($\eta$ = 33 ml/g, Substitutionsgrad = 1,7) |
| 0,6 g | Hydroxypropylmethylcellulose |
| 0,g | Laurylpyridiniumchlord |
| 96,8 g | Wasser, vollentsalzt |
| 100,0 g | (auf pH 5,0 mit 10 %iger Ameisensäure eingestellt) |

Jeweils 35 g der Haarkurmittel nach Beispiel 12 bzw. 13 wurden im gewaschenen Haar verteilt und nach einer Einwirkungszeit von 3 bis 5 Minuten mit Wasser wieder ausgespült; als Ergebnis wurde ein ausgezeichneter Griff, Glanz sowie Kämmbarkeit des Haares erhalten.

**Beispiel 14**

Hautcreme

| | |
|---|---|
| 0,30 g | Glyceryl-Chitosan ($\eta$, = 33 ml/g, Substitutionsgrad = 1,7) |
| 3,00 g | Stearylalkohol |
| 1,00 g | Wollfettalkohol (adeps lanae) |
| 1,00 g | Vaseline |
| 0,76 g | Milchsäure, 10 %ig |
| 1,00 g | Natriumcetylstearylsulfat |
| 92,94 g | Wasser, vollentsalzt |
| 100,00 g | |

**Beispiel 15**

Haartönungsmittel

| | |
|---|---|
| 0,50 g | Glyceryl-Chitosan ($\eta$ = 30 ml/g, Substitutionsgrad = 1,44) |
| 12,00 g | Cetylstearylalkohol |
| 0,10 g | Parahydroxybenzoesäureäthylester |
| 6,00 g | Laurylalkohol-diglycoläthersulfat-Natriumsalz 28 %ige väßrige Lösung) |
| 0,50 g | Parfümöl |
| 79,31 g | Wasser |
| 0,50 g | 1-Hydroxy-2-amino-4-nitrobenzol (C.I. 76 530) |
| 0,85 g | 1,4-Diamino-2-nitrobenzol (C.I. 76 070) |
| 0,24 g | Natriumhydroxyd |
| 100,00 g | |

Ungefähr 30 bis 40 g wurden in dem gewaschenen Haar verteilt und nach einer Einwirkungszeit von ca. 20 Minuten ausgespült. Das Haar war rötlich gefärbt und wies eine gute Kämmbarkeit und einen angenehmen Griff auf.

**Beispiel 16**

<u>Oxidationshaarfärbemittel</u>

| | |
|---|---|
| 0,50 g | Glyceryl-Chitosan (η = 33 ml/g, Substitutionsgrad = 1,7) |
| 0,08 g | 3,5-Diamino-2,6-dimethoxypyridin-Dihydrochlorid |
| 0,30 g | 1,4-Diaminobenzol |
| 0,25 g | Resorcin |
| 0,30 g | Natriumsulfit |
| 3,50 g | Laurylalkohol-diglycoläthersulfat-Natriumsalz 28 %ige wäßrige Lösung) |
| 15,00 g | Cetylalkohol |
| 3,00 g | Ammoniak |
| 77,07 g | Wasser |
| 100,00 g | |

50 g dieses Haarfärbemittels wurden mit 50 ml 6 %iger Wasserstoffperoxidlösung gemischt und auf weißes Haar aufgetragen. Nach 30 Minuten wurde das Haar mit Wasser ausgespült und getrocknet. Das Haar hatte eine natürlich wirkende matt-blonde Färbung sowie einen natürlichen angenehmen Griff.

**Beispiel 17**

<u>Dauerwellmittel</u>

| | |
|---|---|
| 0,5 g | Glyceryl-Chitosan (η = 203 ml/g, Substitutionsgrad = 3,1) |
| 10,0 g | Thioglykolsäure |
| 8,0 g | Ammoniak, 25 %ig |
| 6,1 g | Ammoniumhydrogencarbonat |
| 75,4 g | Wasser |
| 100,0 g | |

Zur Anwendung trug man dieses Dauerwellmittel auf das gewickelte handtuchtrockene Haar gleichmäßig auf und ließ es etwa 20 Minuten einwirken; danach wurde das Haar mit Wasser ausgespült und in bekannter Weise oxidativ behandelt. Es wurde ein gutes Wellergebnis erhalten, die Haare fühlten sich natürlich und weich an.

**Patentansprüche**

1. Kosmetisches Mittel zur Behandlung von Haaren oder der Haut, <u>gekennzeichnet</u> durch einen Gehalt an Glyceryl-Chitosan, und zwar makromolekularen, vom Chitosan abgeleiteten Verbindungen, bestehend aus:
a) 4-40 Mol% Einheiten der Formel (I)

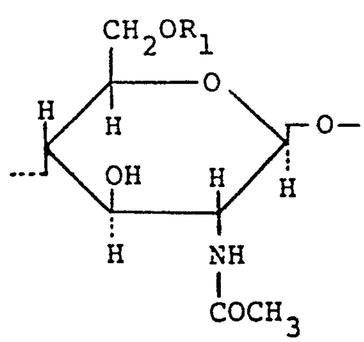

und
b) 60-96 Mol% Einheiten der Formel (II)

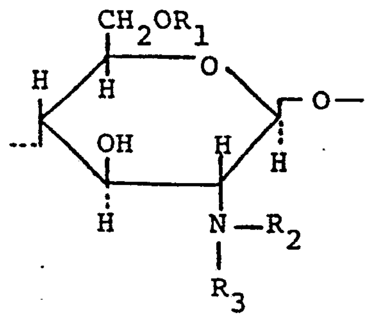

- worin $R_1$, $R_2$ und $R_3$ gleich oder verschieden sind und

—H ,

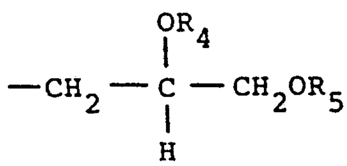

oder

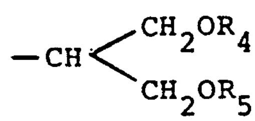

darstellen, wobei $R_4$ und $R_5$ gleich oder verschieden sind und die Substituenten

—H ,

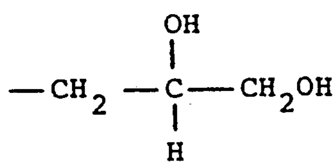

,

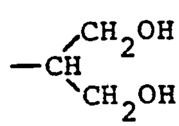

oder

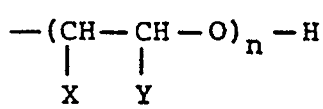

darstellen worin entweder X=H und Y=$CH_2OH$ bedeuten oder Y=H und X=$CH_2OH$ bedeuten und n = 2 bis 5 ist, mit der Maßgabe, daß bei mindestens der Hälfte der Einheiten der Formel II $R_1$, $R_2$ und $R_3$ nicht gleichzeitig H sind, bzw. dessen wasserlöslichen Salze mit organischen oder anorganischen Säuren.

2. Mittel nach Anspruch 1, gekennzeichnet durch einen Gehalt an Glyceryl-Chitosan von 0,05 bis 10 Gewichtsprozent.

3. Mittel nach Anspruch 1 und 2, dadurch gekennzeichnet, daß es ein Haarwaschmittel ist.

4. Mittel nach Anspruch 1 bis 3, dadurch gekennzeichnet, daß es ein anionisches oberflächenaktives Agens enthält.

5. Mittel nach Anspruch 1 bis 4, dadurch gekennzeichnet, daß das anionische oberflächenaktive Agens ein Alkyläthersulfat ist.

6. Mittel nach Anspruch 1 und 2, dadurch gekennzeichnet, daß es ein Mittel zur Festigung der Frisur ist.

7. Makromolekulare, vom Chitosan abgeleitete Verbindungen bestehend aus

a) 4-40 Mol% Einheiten der Formel (I)

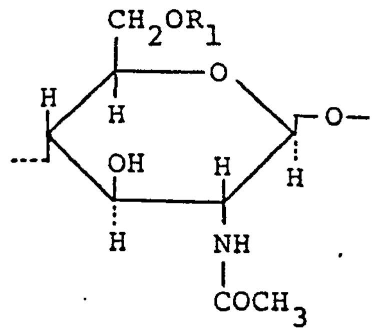

,

und

b) 60-96 Mol% Einheiten der Formel (II)

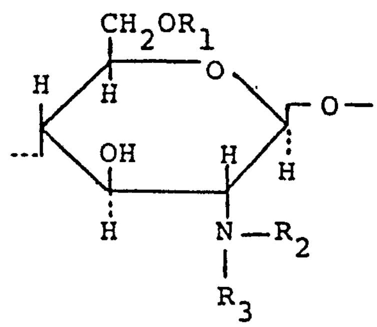

wobei $R_1$, $R_2$ und $R_3$ gleich oder verschieden sind und

—H ,

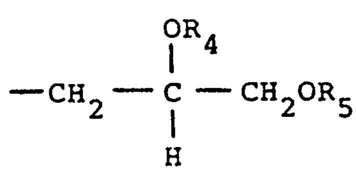

oder

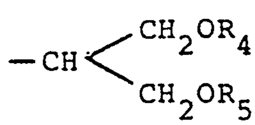

darstellen, wobei $R_4$ und $R_5$ gleich oder verschieden sind und die Substituenten

$-H$ ,

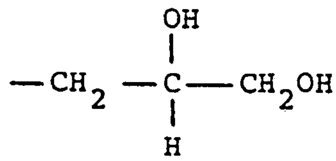 ,

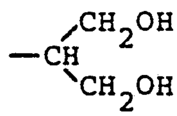

oder

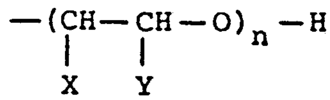

darstellen wobei entweder X=H und Y=$CH_2OH$ bedeuten oder Y=H und X=$CH_2OH$ bedeuten und n = 2 bis 5 ist, mit der Maßgabe, daß bei mindestens der Hälfte der Einheiten der Formel II $R_2$ und $R_3$ nicht gleichzeitig H sind, sowie deren wasserlöslichen Salze mit organischen oder anorganischen Säuren.

8. Verfahren zur Herstellung der Verbindungen nach Anspruch 7, dadurch gekennzeichnet, daß man ein Chitosan, bestehend aus zu 60 - 96 % entacetyliertem Chitin, mit Glycidol (1,2-Epoxypropanol-3) in geeignetem Verhältnis zur Umsetzung bringt.

9. Verfahren nach Anspruch 8, dadurch gekennzeichnet, daß man das Chitosan mit oder ohne Lösungsmittel mit Glycidol vermischt und diese Mischung bei einer Temperatur zwischen 10 und 100°C mehrere Stunden mischt oder rührt.

10. Verfahren nach den Ansprüchen 8 und 9, dadurch gekennzeichnet, daß die Umsetzung in Gegenwart von Wasser durchgeführt wird.

11. Verfahren nach einem der Ansprüche 8 bis 10, dadurch gekennzeichnet, daß bei der Umsetzung zusätzlich organische oder anorganische Säuren oder Basen zugesetzt werden.

12. Verfahren nach den Ansprüchen 8 bis 11, dadurch gekennzeichnet, daß als Ausgangsmaterial durch Umfällen und Tiefgefrieren strukturell modifiziertes Chitosan eingesetzt wird.

**Claims**

1. Cosmetic composition for hair or skin treatment, characterised by a content of glycerol-chitosan and in fact of macromolecular compounds derived from chitosan, consisting of:

a) 4 to 40% mole units of formula (I)

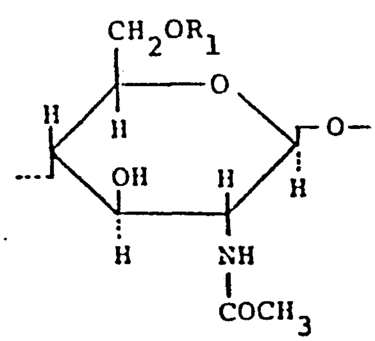

and

b) 60 to 96% mole units of formula (II)

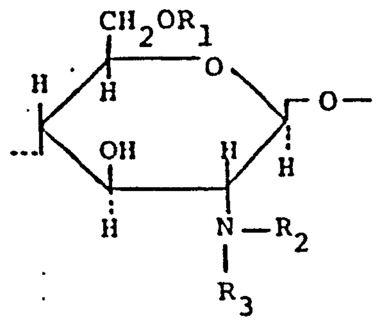

-wherein $R_1$, $R_2$ and $R_3$ are the same or different and represent

—H ,

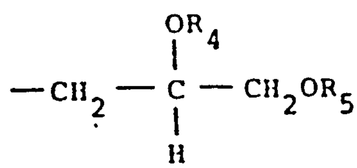

or

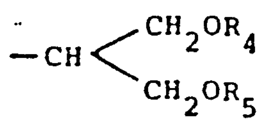

$R_4$ and $R_5$ being the same or different and representing the substituents

—H ,

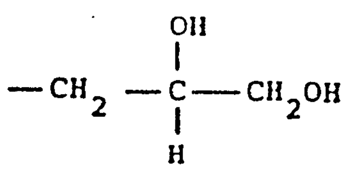

,

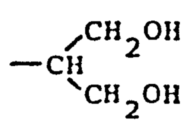

or

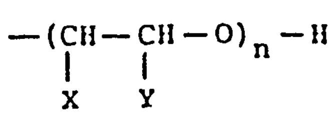

in which either X=H and Y=$CH_2OH$ or Y=H and X=$CH_2OH$ and n = 2 to 5, with the proviso that for at least half of the units to formula II $R_1$, $R_2$ and $R_3$ are not all H at the same time, or their water soluble salts with organic or inorganic acids.

2. Composition according to Claim 1, characterised by a content of 0.05 to 10% by weight of glycerol-chitosan.

3. Composition according to Claim 1 and 2, characterised in that it is a hair washing composition.

4. Composition according to Claim 1 to 3, characterised in that it contains an anionic surface active agent.

5. Composition according to Claim 1 to 4, characterised in that the anionic surface active agent is an alkyl ether sulphate.

6. Composition according to Claim 1 and 2, characterised in that it is a medium for setting a hair style.

7. Macromolecular compounds derived from chitosan and consisting of

a) 4 to 40% mole units of formula (I)

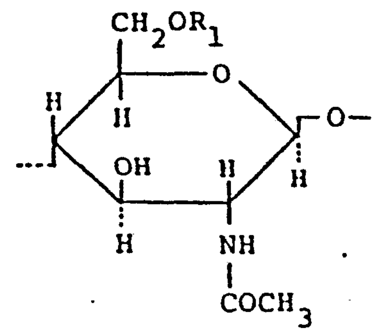

and

b) 60 to 96% mole units to formula (II)

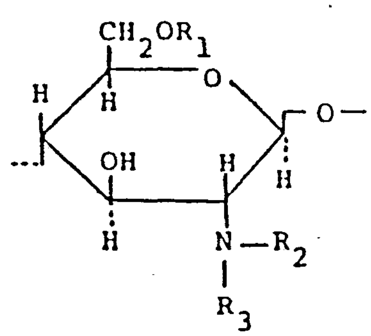

in which $R_1$, $R_2$ and $R_3$ are the same or different and represent

—H ,

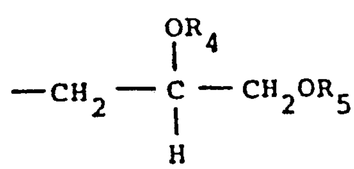

or

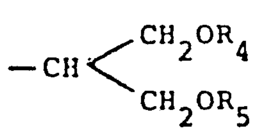

$R_4$ and $R_5$ being the same or different and representing the substituents

—H ,

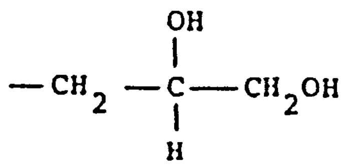 ,

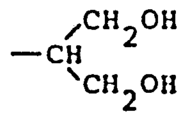

or

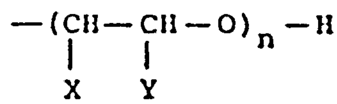

where either X=H and Y=$CH_2OH$ or Y=H and X=$CH_2OH$ and n = 2 to 5, with the proviso that for at least half of the units of formula II $R_2$ and $R_3$ are not simultaneously H, or their water soluble salts with organic or inorganic acids.

8. Method of producing the compounds according to Claim 7, characterised in that a chitosan consisting of 60 to 96% deacetylated chitin is caused to react in a suitable ratio with glycidol (1,2-epoxy propanol-3).

9. Method according to Claim 8, characterised in that the chitosan, with or without a solvent, is mixed with glycidol, the mixture being mixed or agitated for several hours at a temperature of between 10 and 100°C.

10. Method according to Claims 8 and 9, characterised in that the reaction is carried out in the presence of water.

11. Method according to one of Claims 8 to 10, characterised in that organic or inorganic acids or bases are additionally added for the reaction.

12. Method according to Claims 8 to 11, characterised in that the starting material used is chitosan which has been structurally modified by reprecipitation and deep freezing.

**Revendications**

1. - Produit cosmétique pour le traitement des cheveux ou de la peau, caractérisé en ce qu'il renferme du glycéryl-chitosane, c'est à dire des composés macromoléculaires dérivant du chitosane, comprenant:

a) un pourcentage molaire de 4 à 40 % de groupes répondant à la formule (I) > > >

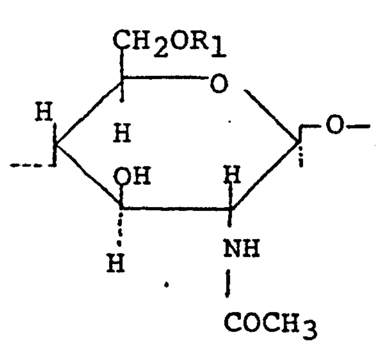

b) un pourcentage molaire de 60 à 96 % de groupes répondant à la formule (II)

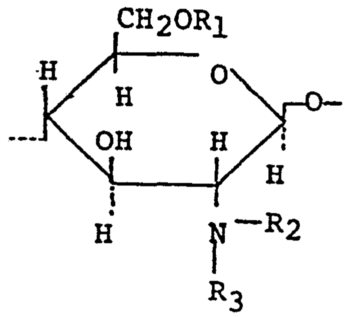

où $R_1$ $R_2$ et $R_3$ sont identiques ou différents et représentent

— H ,

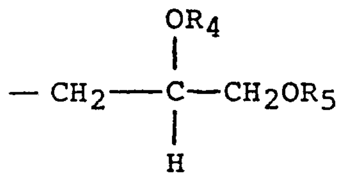

ou

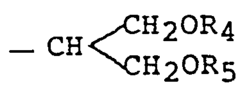

$R_4$ et $R_5$ étant identiques ou différents et représentant les substituants

— H , 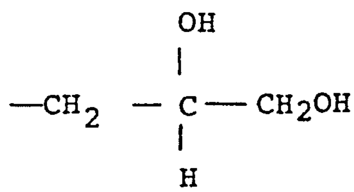 , 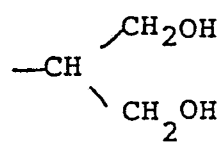

ou

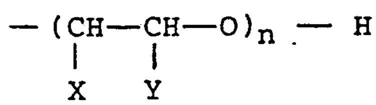

où X=H et Y=$CH_2OH$ ou bien Y=H et X=$CH_2OH$ et n = 2 à 5, avec comme condition déterminante que, pour au moins la moitié des groupes répondant à la formule II, $R_1$, $R_2$ et $R_3$ ne représentent pas en même temps H, ou leurs sels solubles dans l'eau avec des acides organiques ou inorganiques.

2. - Produit selon la revendication 1, caractérisé par une teneur en glycéryl-chitosane de 0,05 à 10 % en poids.

3. - Produit selon les revendications 1 et 2, caractérisé en ce que c'est un produit de lavage des cheveux.

4. - Produit selon les revendications 1 à 3, caractérisé en ce qu'il renferme un agent tensio-actif anionique.

5. - Produit selon les revendications 1 à 4, caractérisé en ce que l'agent tensio-actif anionique est un éther-sulfate d'alkyle.

6. - Produit selon les revendications 1 et 2, caractérisé en ce que c'est un produit de fixation de la coiffure.

7. - Composés macromoléculaires dérivant du chitosane, comprenant

a) un pourcentage molaire de 4 à 40 % de groupes répondant à la formule (I)

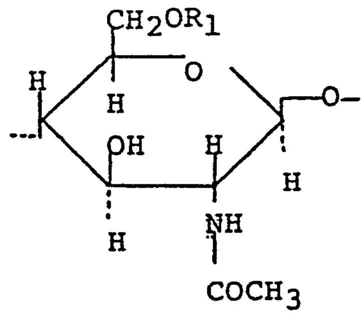

et

b) un pourcentage molaire de 60 à 96 % de groupes répondant à la formule (II)

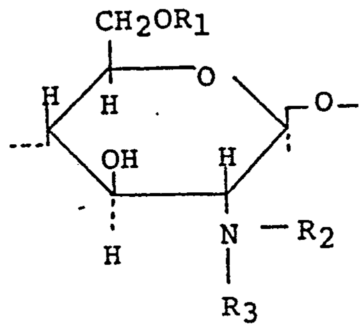

$R_1$, $R_2$ et $R_3$ étant identiques ou différents et représentant

$-H$ ,

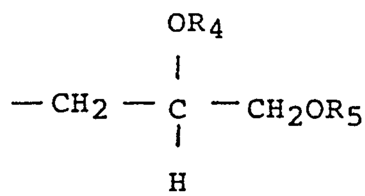

ou

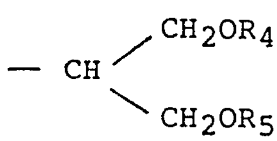

$R_4$ et $R_5$ étant identiques ou différents et représentant les substituants

$-H$ ,

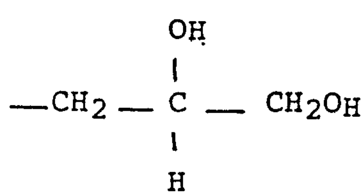

,

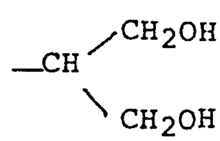

ou

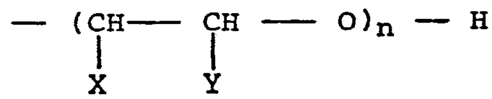

X=H et Y=$CH_2OH$ ou bien Y=H et X=$CH_2OH$, et n = 2 à 5, avec pour condition déterminante que, pour au moins la moitié des groupes répondant à la formule II, $R_2$ et $R_3$ ne représentent pas en même temps H, ainsi que leurs sels solubles dans l'eau avec des acides organiques ou inorganiques.

8. - Procédé de préparation des composés selon la revendication 7, caractérisé en ce qu'on fait réagir un chitosane comprenant 60 à 96 % de chitine désacétylée sur du glycidol (1,2-époxypropanol-3) dans une proportion appropriée.

9. - Procédé selon la revendication 8, caractérisé en ce qu'on mélange le chitosane avec ou sans solvant avec du glycidol et l'on mélange ou l'on agite ce mélange plusieurs heures à une température comprise entre 10 et 100° C.

10. - Procédé selon les revendications 8 et 9 caractérisé en ce qu'on effectue la réaction en préseence d'eau.

11. - Procédé selon l'une des revendications 8 à 10, caractérisé en ce que, lors de la réaction, on ajoute en plus des acides ou des bases organiques ou inorganiques.

12. - Procédé selon les revendications 8 à 11, caractérisé en ce qu'on utilise, comme matière de départ, du chitosane à structure modifiée par reprécipitation et congélation.